Europäisches Patentamt

European Patent Office

Office européen des brevets

(19)

(11) Veröffentlichungsnummer : **0 351 885 B1**

(12) **EUROPÄISCHE PATENTSCHRIFT**

(45) Veröffentlichungstag der Patentschrift :
**27.01.93 Patentblatt 93/04**

(51) Int. Cl.⁵ : **A61K 31/52**

(21) Anmeldenummer : **89114991.6**

(22) Anmeldetag : **12.07.85**

(54) Verwendung von Pentoxifyllin zur Behandlung entzündlicher Erkrankungen.

(30) Priorität : **21.07.84 DE 3426961**
**07.03.85 DE 3508097**

(43) Veröffentlichungstag der Anmeldung :
**24.01.90 Patentblatt 90/04**

(45) Bekanntmachung des Hinweises auf die
Patenterteilung :
**27.01.93 Patentblatt 93/04**

(84) Benannte Vertragsstaaten :
**AT BE CH DE FR GB IT LI LU NL SE**

(56) Entgegenhaltungen :
**EP-A- 0 069 958**
**BIOLOGICAL ABSTRACTS/RRM-BIOSIS, Nr.**
**27012246, Philadelphia, Pennsylvania, US; M.**
**SAKURAI et al.: "Effect of 1-5'oxohexyl-3-**
**methyl-7-propyl xanthine HWA-285 on tri ethyl**
**tin induced brain edema in rats 1. Effect on**
**brain wet weight and watercontents"**

(56) Entgegenhaltungen :
**BIOLOGICAL ABSTRACTS/RRM-BIOSIS, Nr.**
**12070171, Philadelphia, Pennsylvania, US; J.**
**REMIEN et al.: "The influence of vasoactive-**
**and anti inflammatory agents on red blood cell**
**deformability and blood viscosity"**
**"Rote Liste", 1987, Nr. 36056, Editio Cantor,**
**Aulendorf, DE**
**BR. J. PHARMAC., Band 81, 1984, Seiten**
**575-581, The Macmillan Press Ltd, London,**
**GB; W. GALLENKÄMPER et**
**al.:"Cardioprotective actions of pentoxifylline**
**in an animal model of acute myocardial**
**ischaemia"**

(60) Veröffentlichungsnummer der früheren
Anmeldung nach Art. 76 EPü : **0 169 466**

(73) Patentinhaber : HOECHST
AKTIENGESELLSCHAFT
Postfach 80 03 20
W-6230 Frankfurt am Main 80 (DE)

(72) Erfinder : Weithmann, Klaus Ulrich, Dr.
Am Domherrenwald 18
W-6238 Hofheim am Taunus (DE)
Erfinder : Seiffge, Dirk, Dr.
Hauptstrasse 2
W-6309 Münzenberg (DE)

EP 0 351 885 B1

**Beschreibung**

Das 1-(5-Oxohexyl)-3,7-dimethylxanthin (Pentoxifyllin) wird bekanntlich als pharmazeutisches Mittel zur Verbesserung der Fließfähigkeit des Blutes eingesetzt. Als Ursache hierfür wird angenommen (Deutsche Mediz. Wochenschrift 107 (1982), 1674), daß die Fließschubspannung des Blutes abnimmt, weil die Verformbarkeit der Erythrozyten durch Pentoxifyllin-Therapie verbessert wird.

Es wurde nun gefunden, daß eine Anwendung von Pentoxifyllin bzw. deren pharmakologisch verträglichen Salzen eine starke Verbesserung der Therapie von Krankheiten ermöglicht, die durch gestörte Leukocyten verursacht werden.

Gegenstand der Erfindung sind daher Präparate, enthaltend Pentoxifyllin (s. Patentanspruch 1) bzw. deren pharmakologisch verträgliche Salze mit oder ohne einen pharmazeutischen Träger zur Anwendung in der Therapie von Krankheiten, die durch gestörte Leukocyten verursacht werden. Mit anderen Worten eignen sich die erfindungsgemäßen Mittel aufgrund ihrer Effekte zur entzündungshemmenden, schmerzstillenden und cytostatischen Therapie bzw. Prophylaxe. Gegenstand der Erfindung ist daher auch die Verwendung von Pentoxifyllin mit oder ohne pharmazeutische Träger zur Herstellung von Mitteln gegen Krankheioten, die durch gestörte Leukocyten verursacht werden.

Die als Entzündungshemmer bereits bekannte Acetylsalicylsäure kann in den üblichen Dosierungen unerwünschte Nebenwirkungen (z.B. British Journal of Clinical Pharmacology 1980, 10, Suppl. 2 und International Meeting on Side Effects of Antiinflammatory, Analgesic Drugs, Verona, 13.-15.9.82, Abstracts) wie Asthma, allergische Urtikaria, analgetische Nephropathie sowie Magen- und Darmulcera hervorrufen.

Die Dosiseinheiten können in Form von festen Arzneiformen, wie Kapseln (einschließlich Mikrokapseln), die im allgemeinen keine pharmazeutischen Träger enthalten), Tabletten (einschließlich Dragees und Pillen), oder Zäpfchen vorliegen, wobei bei Verwendung von Kapseln das Kapselmaterial die Funktion des Trägers wahrnehmen und der Inhalt z.B. als Pulver, Gel, Emulsion, Dispersion, Lösung vorliegen kann. Besonders vorteilhaft und einfach ist es jedoch, orale und perorale Formulierungen mit dem Wirkstoff herzustellen, die die berechneten Mengen des Wirkstoffes zusammen mit jedem gewünschten pharmazeutischen Träger enthalten. Auch eine entsprechende Formulierung (Zäpfchen) für die rektale Therapie kann angewandt werden. Ebenso ist die transdermale und parenterale (intraperitoneale, intravenöse, subkutane, intramuskuläre) Injektion von Lösungen, z.B. mittels geeigneter Injektionseinheiten möglich.

Derartige Präparate können nach üblichen Verfahren hergestellt werden, indem man in üblicher Weise, z.B. nach Sucker, Fuchs und Speiser, Pharmazeutische Technologie, Stuttgart 1978, S. 424, Tabletten, Pillen oder Granulatkörper, die als Arzneimittel Pentoxifyllin enthalten herstellt, vorteilhaft in Kombination mit Schleimbildnern, Harzen wie Polystyrol, oder anderen üblichen, die Verträglichkeit fördernden Mitteln. Die Tabletten, Pillen oder Granulatkörper können nach üblichen Verfahren hergestellt werden und enthalten Trägermittel und andere übliche Hilfsstoffe, wie Stärke, z.B. Kartoffel-, Mais- oder Weizenstärke, Zellulose bzw. deren Derivate, insbesondere mikrokristalline Zellulose, Siliziumdioxid, verschiedene Zucker wie Milchzucker, Magnesiumcarbonat und/oder Kalziumphosphate. Pentoxifyllin kann z.B. mit Hilfe der in der pharmazeutischen Technologie üblichen Verfahren, wie Preß-, Tauch- oder Wirbelbettverfahren, oder durch Kesseldragierung aufgezogen werden. Die Dragierlösung besteht gewöhnlich aus Zucker- und/oder Stärkesirup, unter Zusatz von Gelatine, Gummi arabicum, Polyvinylpyrrolidon, synthetischen Zelluloseestern, oberflächenaktiven Substanzen, Weichmachern, Pigmenten und ähnlichen Zusätzen entsprechend dem Stand der Technik.

Zur Herstellung der Arzneiformen kann jedes der üblichen Fließregulierungs-, Schmier- bzw. Gleitmittel wie Magnesiumstearat und Trennmittel verwendet werden.

Die anzuwendende Dosierung ist selbstverständlich abhängig von verschiedenen Faktoren, wie dem zu behandelnden Lebewesen (d.h. Menschen oder Tieres, Alter, Gewicht, allgemeiner Gesundheitszustand), der Schwere der Symptome, der zu be handelnden Erkrankung, (falls vorhanden) der Art der Begleitenden Behandlung mit anderen Arzneimitteln, der Häufigkeit der Behandlung usw. Die Dosierungen werden im allgemeinen bis zu fünfmal pro Tag und vorzugsweise einmal bis dreimal pro Tag verabreicht. Die Menge des Pentoxifyllins soll innerhalb des für das zu behandelnde Lebewesen verträglichen, wirksamen Dosierungsbereichs liegen.

Die bevorzugte Dosis Pentoxifyllin beträgt beim Menschen bei alleiniger Verabreichung zwei- bis dreimal täglich 200 bis 800, insbesondere 300 mg bis 600 mg. Eine geeignete Therapie besteht somit z.B. in der Verabreichung von einer, zwei oder mehr, vorzugsweise 3 bis 8 Einzeldosierungen des erfindungsgemäßen Präparates von je 100 bis 600, vorzugsweise mindestens 200 und insbesondere bis 400 mg Pentoxifyllin von der Zahl der Einzeldosierungen und auch der zu behandelnden Krankheit abhängig ist und eine Einzeldosierung z.B. auch aus mehreren gleichzeitig verabreichten Tabletten bestehen kann.

2

In vivo Untersuchungen

Die Testsubstanzen wurden in 0,9% Natriumchlorid-Lösung (enthielt 1% Carboxymethylzellulose (Serva, Heidelberg) per os appliziert. Kontrolltiere wurden in entsprechender Weise, aber ohne Testsubstanzen behandelt. Als Versuchstiere wurden männliche oder weibliche Sprague Dawley- oder Wistar-Ratten verwendet.

Die Untersuchung mit Pentoxifyllin und Acetylsalicylsäuse im Laser-induzierten Thrombosemodell erfolgte an weiblichen Sprague-Dawley-Ratten mit einem Körpergewicht von ca. 200 g. Die zu untersuchenden Tiere wurden mit 0.1 mg Atropinum suf. sol. s.c. prämediziert und mit 100 mg Ketaminhydrochloride und 4 mg Xylazin pro kg KG i.p. anaesthetisiert. Zur Untersuchung dienten Arteriolen und Venolen des mit entgastem Paraffinöl überschichteten Mesenteriums mit einem Durchmesser von ca. 13 μm. Der Strahl des 4 W Argon-Lasers (Fa. Spectra Physics, Darmstadt,) wurde mittels einer Strahladaptions- und -justieranlage (Fa. BTG, München,) koaxial in den invertierten Strahlengang eines Mikroskopes (ICM 405, LD-Epipland 40/0.60; Fa. Zeiss, Oberkochen,) eingebracht. Die benutzte Wellenlänge beträgt 514,5 nm mit einer Energie oberhalb des Objektives von 30.5 mW. Die Expositionszeit pro Einzelschuß dauert 1/15 sec. Alle Meßvorgänge wurden per Videokamera (Trinicon-Röhre, Sony, Köln) aufgenommen und auf einem Rekorder (Sony, U-matic 3/4″) gespeichert. Die Testsubstanzen wurden den Versuchstieren in verschiedenen Dosierungen oral eine Stunde, bei i.v. Gabe 10 min vor Versuchsbeginn verabreicht, Kontrolltiere erhielten die gleiche Menge des Placebos. Die Applikation der Substanzen erfolgte als Einzelgabe.

Auswertung:

Es wird die Zahl der Schüsse gezählt, um einen definierten Thrombus zu induzieren. Die Schußfrequenz beträgt eine Läsion alle 2 min, wobei alle während des Beobachtungszeitraumes gebildeten Thromben von einer Mindestgröße von 1/4 Gefäßradius ausgezählt und vermessen wurden.

Die statistische Auswertung der Versuchsergebnisse erfolgte mit Hilfe des $\chi^2$-Testes (L. Cavalli-Sforza, Biometrie, Stuttgart, 1969, S.49 ff).

Ex vivo Untersuchungen:

Die Thrombozytenaggregation wurde nach an sich bekannten Verfahren bestimmt. Männliche Kaninchen (eigene Zucht, BASK, SPF Wiga ca. 2,5 bis 3,5 kg) wurden intravenös (Ohrvene) mit Pentoxifyllin, gelöst in physiologischer Kochsalzlösung, behandelt. Hierauf wurde Blut aus der Ohrvene gezogen, mit einer 3,8%igen Trinatriumzitratlösung im Verhältnis 9 : 1 versetzt und 45 Minuten bei Zimmertemperatur inkubiert. Sodann wurde 10 Minuten bei 1000 Umdrehungen pro Minute zentrifugiert. Die obere Schicht, die das plättchenreiche Plasma bildet, wurde abgetrennt und die untere Schicht 10 Minuten bei 28 000 Umdrehungen pro Minute zentrifugiert. Nun enthielt die obere Schicht das plättchenarme Plasma, das ebenfalls abgetrennt wurde. Das plättchenreiche Plasma wurde mit Hilfe des plättchenarmen Plasma auf etwa 6 bis 7·10⁸ Plättchen/ml verdünnt (Coulter Counter, Coulter Electronics, Krefeld). Die Plättchenaggregation wurde optisch durch Messung der Lichttransmission in einem Born'schen Aggregometer (Fa. Labor GmbH, Hamburg) verfolgt. Das Volumen des Testansatzes war 0,25 ml, die Temperatur 37°C. Die Aggregationsinduktion erfolgte mit $2 \cdot 10^{-4}$M Arachidonsäure (Serva, Heidelberg), gereinigt unter Schutzgas (Argon) über präparative Hochdruckflüssigkeitschromatogaphie (HPLC Reversed Phase C-18-Säule). Die Zunahme der Thrombozytenaggregation wurde an Hand der Lichttransmission verfolgt. Meßgröße in diesem System ist die maximale Aggregationsamplitude E.

Untersuchungen im Modell der chronischen Entzündung

Pentoxifyllin wurde im pathologischen Rattenmodell der Adjuvans-Arthritits (induziert mit Mycobacterium butyricum) gemäß Clinical Hemorheology 3 (1983) 469-480 hinsichtlich ihrer blutrheologischen, antithrombotischen, antiaggregatorischen und antiinflammatorischen Wirkung nach 21tägiger oraler Gabe untersucht. Die Blutentnahme aus der thorakalen Aorta erfolgte 1 Stunde nach der letzten Substanzapplikation. Die blutrheologische Wirkung wurde in allen Einzelheiten bestimmt wie in Clinical Hemorheology 4 (1984) 263-273 beschrieben. Es wurde die Erythrozytenverformbarkeit im Filtrometer (MF 4, Fa. Myrenne, Roetgen, Deutschland) durch Auswertung der initialen Steigerung der Fleißkurve quantifiziert. Tab. 1 zeigt, daß die, verglichen mit gesunden Kontrollratten, in Arthritisratten herabgesetzte Erythrozytenfiltrierbarkeit durch Acetylsalicylsäure und Pentoxifyllin wieder gesteigert werden kann.

Die antithrombotische Wirkung wurde im Laser-Modell gemessen, wie oben beschrieben. Tab. 1 zeigt die Ergebnisse. Während in den gesunden Kontrolltieren durchschnittlich 2,173 (=100%) Laserschüsse zur Erzielung eines Thrombus gesetzt werden müssen, reichen im Arthritis-Tier 0,99 (=46%) Schüsse aus, d.h. im kranken Tier ist die Thromboseneigung erhöht. Tab. 1 zeigt, daß die Thromboseneigung durch die Behandlung mit den Medikamenten herabgesetzt wird und insbesondere durch Anwendung von Pentoxifyllin den im gesunden Tier gefundenen Werten angeglichen wird.

Die Messungen zur Thrombozytenaggregation erfolgten wie oben ausführlich beschrieben. Statt Arachidonsäure wurde jedoch 0,04 mg Collagen zur Induktion der Thrombozytenaggregation in 1 ml plättchenreichem Plasma (Aggregometer PA II, Fa. Myrenne, Roetgen) verwendet. Die Aggregationsamplitude (Aggregationsneigung) bei der unbehandelten arthritischen Ratte ist am höchsten (=100%), bei der gesunden Ratte tritt mit 0,04 mg Collagen keine Aggregation auf (Tab. 1). Die aufgelisteten Ergebnisse zeigen, daß die pathologisch gesteigerte Aggregationsneigung in der arthritischen Ratte durch Pentoxifyllin vermindert werden kann.

Die antiinflammatorische Wirkung wurde, wie oben zitiert, an Hand des Volumens des Pfotenoedems und des Standard-Nekroseindex quantifiziert. Unter Behandlung mit Pentoxifyll nimmt die Nekrose- und Oedembildung deutlich ab. Tab. 2 zeigt die relative Besserung der Symptome in den behandelten Tieren, verglichen mit den unbehandelten Arthritis-Ratten.

Tabelle 1

Einfluß verschiedener Medikamente auf Blutparameter der arthritischen Ratte
(n = 8 pro Gruppe) (Einzelheiten siehe Text)

| Arthritisratte | Erythrozyten-filtrierbarkeit | Thrombose-neigung (Anzahl der Schüsse) | induz. Thrombozyten-aggregation |
|---|---|---|---|
| unbehandelt | 59 % | 46 % | 100 % |
| 10 mg Acetylsalicylsäure | 63 % | 47 % | 62 % |
| 180 mg Acetylsalicylsäure | 68 % | 91 % | 35 % |
| 30 mg Pentoxifyllin | 79 % | 87 % | 94 % |
| (gesunde Kontrollratten | 100 % | 100 % | 0 % ) |

Tabelle 2

Prozentuale Verbesserung der mit den verschiedenen Medikamenten behandelten
arthritischen Ratten im Vergleich zu unbehandelten arthritischen Ratten
(n = 8 pro Gruppe)
(Einzelheiten siehe Text)

| | Pfotenoedem-Volumen | | Nekrose-Index |
|---|---|---|---|
| | linke | rechte | |
| | Pfote | | |
| 10 mg Acetylsalicylsäure | 2 | 3 | 8 |
| 180 mg Acetylsalicylsäure | 28 | 26 | 28 |
| 30 mg Pentoxifyllin | 3 | 0 | 7 |

**Patentansprüche**

1. Verwendung von Pentoxifyllin = 1-(5-Oxohexyl)-3,7-dimethylxanthin zur Herstellung eines Arzneimittels zur Behandlung entzündlicher Erkrankungen.

**Claims**

1. The use of pentoxifylline = 1-(5-oxohexyl)-3,7-dimethyl xanthine for the preparation of a pharmaceutical for the treatment of inflammatory disorders.

**Revendications**

1. Utilisation de pentoxifylline = 1-(5-oxohexyl)-3,7-diméthylxanthine, pour fabriquer un médicament destiné au traitement de maladies inflammatoires.